Europäisches Patentamt

European Patent Office

Office européen des brevets

(19)

(11) Publication number: **0 335 133**
**A2**

(12) # EUROPEAN PATENT APPLICATION

(21) Application number: **89103858.0**

(51) Int. Cl.⁴: **A61K 47/00**

(22) Date of filing: **06.03.89**

(30) Priority: **31.03.88 US 175741**

(43) Date of publication of application:
**04.10.89 Bulletin 89/40**

(84) Designated Contracting States:
**BE DE FR GB IT**

(71) Applicant: **ABBOTT LABORATORIES**
**One Abbott Park Road**
**Abbott Park, IL 60064-3500(US)**

(72) Inventor: **Weber, Allan E.**
**508 Liberty Lane**
**Westerville Ohio 43081(US)**

(74) Representative: **Modiano, Guido et al**
**MODIANO, JOSIF, PISANTY & STAUB**
**Modiano & Associati Via Meravigli, 16**
**I-20123 Milan(IT)**

(54) **Drug delivery using pulmonary surfactant fo facilitate absorption.**

(57) Methods and compositions for pulmonary drug delivery include administration to a patient of a drug admixed or covalently bonded to a component of a surfactant protein and phospholipid mixture.

# DRUG DELIVERY USING PULMONARY SURFACTANT TO FACILITATE ABSORPTION

## Background

The present invention relates in general to the use of surfactants to deliver drugs across mucosal membranes and, in particular, to the use of pulmonary surfactants for the pulmonary delivery of drugs.

The non-invasive systemic administration of drugs is a goal of many pharmaceutical companies. Unfortunately, the transport of drugs through mucosal membranes is complicated by many factors including the lack of suitable vehicles, the hydrophilic or lipophilic characteristics of the drug and the suitability of the intended route of administration (e.g., rectal administration is unpopular).

Traditional methods of drug delivery include oral ingestion and parenteral administration. Drugs may also be delivered through the skin (i.e. transdermally). Pulmonary delivery of drugs is currently used in specialized cases, including the treatment of pulmonary disease with, for example, bronchodilators, and also including the administration of general anesthetics. However, many drugs are not absorbed well through the skin, are not absorbed well from the gastrointestinal tract due to inactivation or due to lack of solubility.

Liposomes, which are unilammelar or multilammelar vesicles having lipid and/or phospholipid entities in a spherical structure dispersed in a vehicle in which they are insoluble, may be used for topical, oral and parenteral drug delivery. In nature, liposomes, or minor modifications thereof, are found in mammalian systems, and may play a role in biological absorption. Examples in nature include the liposomal nature of certain bile salt systems and the liposomal character of chlyomicrons in the intestinal tract.

Therapeutic and diagnostic compositions applied by inhalation and including liposomes are described in Hayward et al., PCT Publication No. WO 87/1586, and Mihalko et al., PCT Publication No. WO 86/06959.

Natural pulmonary surfactant serves the unique role of facilitating the transport of oxygen from the lung to systemic blood flow by reducing the surface tension at the gas-liquid interface in the pulmonary alveoli. Natural pulmonary surfactant is a complex material composed primarily of phospholipids and surfactant-associated proteins or apolipoproteins. The phospholipids, mainly phosphatidycholine ("PC"), disaturated phosphatidycholine ("DSPC") and phosphatidylglycerol ("PG"), are of paramount importance for the physiological role of natural pulmonary surfactant material in reducing surface tension in the alveoli. Phospholipids, of which DSPC is the principal component, are synthesized in the endoplasmic reticulum of Type II epithelial cells in the lung, packaged into lamellar bodies, then secreted into the alveolar space by an exocytotic process. It is believed that several of the phospholipids are not catabolized and resynthesized, but are reutilized after uptake by Type II cells, primarily as intact molecules, and that they constitute the major components of the natural pulmonary surfactant material.

Natural pulmonary surfactant is distinguished from liposomal materials by the inclusion of a series of surfactant-associated proteins or apolipoproteins which are lung-specific. At least three lung-specific proteins have been described. The largest of these proteins has an approximate molecular weight of 30,000-40,000 daltons. This protein, called "SAP-35" or "SP-A," may or may not be required for the pulmonary surfactant function of surface tension reduction at the gas-liquid alveolar interface.

At least two low molecular weight proteins have been identified in natural pulmonary surfactants as being integral parts of the active surfactant. These proteins, called "SAP(Phe)" or "SP-B" and "SAP(Val)" or "SP-C," respectively, are reported to have molecular weights in the range of 3,500-18,000 daltons and to have utility in the reduction of surface tension at the gas-liquid interface. These two proteins have an integral structural function on the disperse phase.

The nature of and the relationship among the various smaller surfactant-associated proteins and the larger protein SAP-35, or its fragments, have not established. Suzuki et al., J. Lipid. Res., 23, 53-61 (1982) suggests that a 15 kd protein may possibly be a physiological regulator for the clearance of alveolar phospholipid.

A small SAP protein isolated from rat alveolar lavage is reported to increase the uptake of liposomes by cultured Type II epithelial cells in Claypool et al., J. Clin. Invest., 74, 677-684 (1984).

The integral nature of the function of SAP-Phe and SAP-Val in pulmonary surfactant is indicated by the inability to demonstrate natural surfactant activity for phospholipids, either singularly or in mixture, in vitro, in vivo or in situ. Likewise, SAP(Phe) and SAP(Val) are not reported to have activity if tested in a similar manner without phospholipids.

Reconstitution of low molecular weight surfactant proteins with synthetic phospholipids imparts virtually complete surfactant-like properties to a mixture, including rapid surface absorption and decreased surface tension during dynamic compression [Yu et al., Biochem. J., 236, 85-89 (1986); and Takahashi et al.,

Biochem. Biophys. Res. Commun., 135, 527-532 (1986)].

Thus, the low molecular weight proteins play a role in the structural functionality related to the reduction of surface tension in the alveoli. Because this is a potential site of drug absorption into the systemic circulation, it is desirable to discover a mixture of phospholipids, proteins, neutral lipids and other pharmaceutical ingredients as a means of carrying drug to the site of absorption and facilitating that absorption to the systemic circulatory system.

## Summary

The present invention provides a drug carrier vehicle for instillation to the lung, especially one composed of a phospholipid-protein or -peptide mixture. The delivery vehicle according to the present invention differs from liposomes in that it is derived from an endogenous pulmonary surfactant isolate or a phospholipid/protein mixture proven to be save or from a derivative thereof. Thus, the materials used to carry drugs according to the present invention are endogenous compounds, the activity and safety of which are known, while liposomal materials are synthetic in nature and the biological response to them may be questioned.

It is also an advantage of the present invention that endogenous materials are believed to be highly conserved, i.e., that the metabolic turnover or clearance from the alveoli is slow. This provides a mechanism for holding a delivered drug at the site of absorption or action for a relatively long period of time.

Another advantage of the present invention is that the high vascularity of lung provides a strong driving force for systemic drug delivery.

Yet another advantage of the present invention is that the protein or peptide and the phospholipid materials provide both the chemical as well as physical "handles" for delivering the drug. One may either exploit covalent attachment of a drug to the peptides, protein or phospholipids or, alternatively, the normal surfactant vesicles may be employed to provide a noncovalent carrier for drugs.

Furthermore, the pulmonary route of administration provided by the present invention offers advantages where other routes of administration have failed or show serious disadvantages. Such advantages include the ability to localize pulmonary therapeutics, such as anti-cancer drugs for lung carcinoma or anti-infectives for the treatment of specific pulmonary infections such as Pneumococcus strains of bacteria related to the disease of pneumonia or for the treatment of opportunistic infections (e.g., Pneumocystis carinii) associated with Acquired Immunodeficiency Disease Syndrome (AIDS) or the AIDS-related complexes (ARC). Similar advantages exist in the use according to the present invention of: drugs which require systemic absorption but which are inactivated or not absorbed when administered orally; drugs which are given chronically via invasive parenteral routes where patient compliance may be in question outside the medical setting; drugs which may be administered via the pulmonary route when the patient is on a respirator; and drugs where a large percentage of first pass metabolism must be avoided to optimize drug efficacy or to avoid side effects attributed to metabolic by-products of the drug.

Therefore, the present invention provides a method for pulmonary drug delivery in which a drug is combined with an admixture of a pulmonary surfactant protein and a phospholipid, and the combined drug, surfactant protein and phospholipid admixture is introduced into a lung of a subject.

The present invention also provides a composition for pulmonary drug delivery including a phospholipid, a pulmonary surfactant protein, and a drug to be administered combined with said protein and phospholipid. According to the present invention, a pulmonary surfactant protein is intended to be interchangable with or to include any pulmonary surfactant peptides, polypeptides, proteins or fragments or analogs thereof as recited herein, as recited in references cited herein or as may come to the attention of those skilled in the art.

This composition may include 50-95% phospholipid, 0-15% neutral lipid (e.g., tripalmatin), 0-15% fatty acids (e.g., palmitic acid), 0.01-10% protein, and 0.1-3% cholesterol, and may further include a phamaceutical excipient.

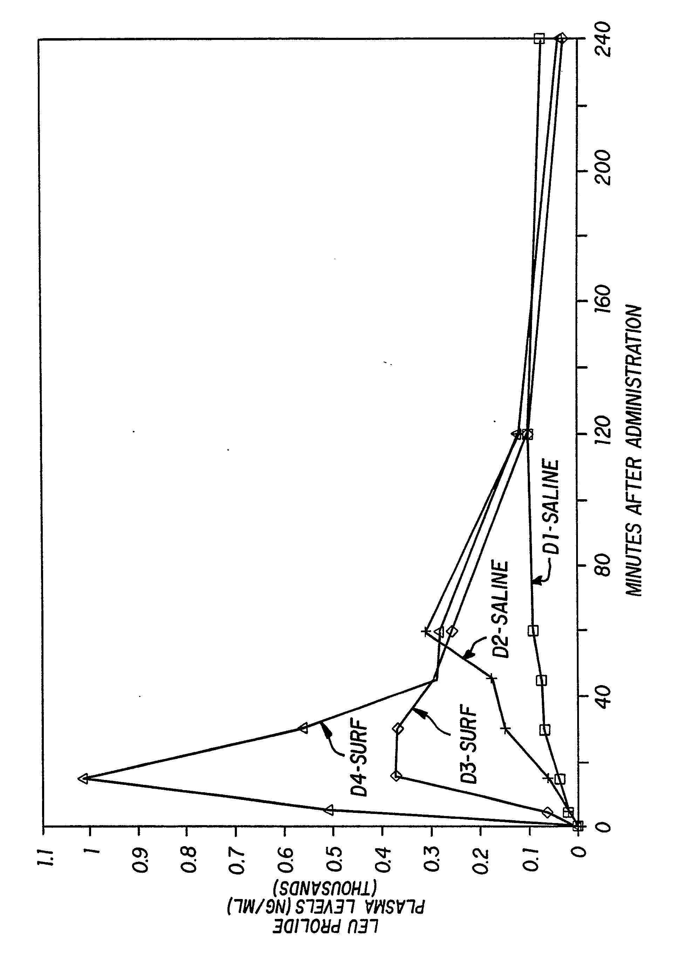

## Brief Description of the Drawing

3

The Figure is a gragh of the results of administration, according to the present invention, of a surfactant-leuprolide composition according to the present invention.

Detailed Description

By either admixture or covalent attachment, a drug or drugs may be incorporated into a phospholipid/peptide or phospholipid/protein mixture which is in the form of a microemulsion or suspension for administration to the lung according to the present invention. The drug may then be either released from the mixture by cleavage of the covalent bond or absorbed directly from the mixture for local or systemic therapeutic function. Covalent attachment of a drug with a reactive group, such as an alcohol or an amine, to a lipid, such as palmitic acid, will yield an ester or amide, respectively. This fatty acid or lipid derivative may be incorporated into the admixture. In the lung or in systemic circulation, non-specific enzymes, such as esterases or amidases, will hydrolyze the derivative to liberate the free drug. Such hydrolysis may also occur by non-enzymatic chemical methods as well. Administration via the lung avoids invasive procedures (injection), destruction in the intestinal tract by local pH or biochemical processes and metabolism due to the first pass effect in the liver.

The proteins or peptides employed according to the present invention may be of bovine or human origin and include peptides derived from bovine and human proteins and analogs thereof. Pulmonary surfactant according to the present invention may be natural surfactant isolated by lavage or extraction of mammalian lung tissue, prepared by admixing synthetic phospholipids and neutral lipids with protein isolates from mammalian sources with proteins prepared by recombinant DNA technology applications or with peptides or proteins prepared identical to or as active analogues of the native proteins by chemical synthetic methods. Proteins and peptides intended for use according to the present invention include all known lung-specific surfactant-associated proteins identified as SAP-35 or alternate terminology, as well as the lower molecular weight proteins variously defined as SAP(Phe), SAP(Val), SP-B, SP-C, SAP-6(Phe), SAP-6(Val), SAP-5, SAP-10, SAP-18 or various other terms but which are specified by the sequences shown in Table 2.

Suitable natural isolate, chemically synthesized and/or recombinant proteins and peptides for use according to the present invention (and isolative and recombinant methods for obtaining them) are disclosed in the following publications which are incorporated by reference herein: Tanaka, U.S. Patent No. 4,338,301; Tanaka, U.S. Patent No. 4,397,839; Takei et al., U.S. Patent No. 4,603,124; Hawgood et al., Proc. Natl. Acad. Sci. (USA), 84, 66-70 (1987); Jacobs et al., J. Biol. Chem., 262, 9808-9811 (1987); Floros et al., J. Biol. Chem., 19, 9029-9033 (1986); Glasser et al., Proc. Natl. Acad. Sci. (USA), 84, 4007-4011 (1987); Whitsett et al., Pediatric Res., 20, 744-749 (1986); Whitsett et al., Pediatric Res., 20, 460-467 (1986); Schilling, Jr. et al., WO87/ 06588; Schilling, Jr. et al., U.S. Patent No. 4,659,805; Taeusch et al., PCT Publication No. WO87/02037; Schilling Jr. et al., PCT Publication No. WO86/03408; Whitsett, PCT Publication No. WO87/06943.

Lipids intended for use according to the present invention include dipalmitylphosphatidyl choline or congeners, tripalmitin, glycerol or phosphate esters thereof, phosphatidyl choline or inositol, palmitic acid or similar compounds and cholesterol in some ratios. It is anticipated that the dose of phospholipid/protein (peptide) may be in the range of 25 to 1000 mg suspended in from 1 to 40 ml of water or saline. The amount of drug carried will depend on the normal dose of the specific drug, its relative bioavailability and its safety.

Drugs which may be delivered by the present invention include those which are sensitive to pH or enzymatic degradation in the GI tract, those not absorbed from the GI tract, those for which an invasive parenteral route is not desirable or possible, those for which it is desirable to be locally administered to the lung or other pulmonary regions, or those for which it is desirable to avoid certain metabolic functions, such as first pass hepatic or GI tract biotransformation. Such formulations may be administered by instillation of the drug product to the pulmonary system or by inhalation, either voluntary or by forced respiration via nebulization or aerosolization with a propellant of fluorcarbons or hydrocarbons, either directly or indirectly.

Example 1

4

Leuprolide, a nonapeptide LHRH agonist, may be incorporated according to the present invention into a surfactant preparation derived from Surfactant TA (Abbott Laboratories, North Chicago, Illinois).

Stock solutions of the various components were prepared as follows:

A. Dipalmitoylphosphatidylcholine (DPPC): Approximately 400 mg of DPPC (Sigma Chemical Co., St. Louis, Missouri) was dissolved in sufficient chloroform:methanol (2:1) to prepare 5 ml of solution at a concentration of approximately 80 mg/ml.

B. Palmitic Acid (PA): Approximately 85 mg of PA (Sigma Chemical Co.) was dissolved in sufficient chloroform:methanol (2:1) to prepare 10 ml of a solution at a concentration of approximately 8.5 mg/ml.

C. Tripalmitin (TP): Approximately 70 mg of TP (Sigma Chemical Co.) was dissolved in sufficient chloroform:methanol (2:1) to prepare 5 ml of a solution at a concentration of approximately 14 mg/ml.

D. Leuprolide: Approximately 50 mg of leuprolide acetate (Abbott Laboratories, North Chicago, Illinois) was dissolved in sufficient methanol to prepare 25 ml of a solution at a concentration of approximately 2 mg/ml as the acetate salt.

An admixture was prepared by mixing stock solutions of DPPC, PA, TP with a chloroform:methanol solution of bovine lung lipids (prepared as described in Tanaka, U.S. Patent No. 4,338,301 and Tanaka, U.S. Patent No. 4,397,839) which are the products of an extraction of bovine lungs to yield a naturally-derived surfactant preparation which is supplemented with the specified phospholipid, neutral lipids, fatty acid material to form an active pulmonary surfactant composition. To the resulting solution, adequate stock solution of the leuprolide acetate was added in the order specified herein. The solvents were evaporated in-vacuo at approximately 40°C. A small quantity of ethanol (approximately 7 ml) was added to the residue followed by evaporation in vacuo at approximately 55°C. Approximately 5 ml of water was added to the residue and concentrated in vacuo at approximately 55°C to remove trace amounts of residual ethanol. The residue was dispersed in 0.9% sodium chloride solution (aqueous) to a final volume of 25 ml. The resulting dispersion was uniformly dispersed by mixing and sonication.

The resulting material was tested biophysically on a Pulsating Bubble Surfactometer ("PBS") and a Wilhelmy Surface Balance ("WSB") and demonstrated excellent surface tension-reducing properties under dynamic conditions which are indicative of an effective pulmonary surfactant. Surface tension minima of 4-6 dynes per square centimeter as determined by either method, PBS or WSB were achieved. The maximum surface tension achieved ranged from 30-42 dynes per square centimeter.

Both 1% and 3% by weight admixtures of leuprolide to total phospholipids were prepared and these admixtures displayed excellent biophysical characteristics. The concentrations correspond to a w/v (weight to volume)composition of the total fluid volume of 250 or 750 μg/ml of leuprolide, respectively.

## Example 2

Stock solutions similar to those described in Example 1 were prepared using DPPC, PA and TP. Vasopressin (8-Arg Vasopressin, Synthetic, Sigma Chemical Co.) was used as supplied at a concentration of 10.3 mg/ml in chloroform. A surfactant preparation was produced as described in Example 1 with the substitution of vasopressin for leuprolide as the drug to be delivered. This admixture were tested on the Pulsating Bubble Surfactometer ("PBS") and surface tension minima of 2-4 dynes/cm$^2$ was achieved. See, Enhorning, S. Appl. Physiol., 43, 198-203 (1978).

## Example 3

To demonstrate that a pulmonary surfactant composition prepared by admixing purified low molecular weight proteins purified by chromatography could be combined with synthetic lipids to produce an active surfactant preparation containing drug for lung delivery, the following sample was prepared.

Stock solutions of DPPC and PA were prepared as follows in Example 1. PG (Sigma Chemical Co.) was obtained as a chloroform solution at a concentration of 10 mg/ml and used as supplied.

Surfactant-associated protein from a silica column preparation described as "SAP-6 proteins" in Whitsett, PCT Publication No. WO 87/06943 (which is incorporated by reference herein), at pages 23-26, was used as a mixture of SAP(Phe) and SAP(Val) in a ratio of approximately 1:2 from a stock solution in chloroform:methanol (2:1) containing approximately 650 micrograms/ml.

The composition of the admixture is disclosed in Table 1.

TABLE 1

| Component | Amount/25 ml | Amount/ml |
|---|---|---|
| DPPC | 425 mg | 17 mg |
| PA | 56.25 mg | 2.25 mg |
| PG | 137.5 mg | 5.5 mg |
| Protein (SAP-6) | 6.25 mg | 0.25 mg |
| Leuprolide acetate | 12.5 mg | 0.5 mg |

A dispersion was prepared as follows: the stock solutions of PG and surfactant-associated protein were mixed and warmed to 45°C. for approximately 1 hour. The solution was cooled to room temperature and the DPPC, PA and leuprolide stock solutions were added. The resulting solution was evaporated in vacuo at approximately 40°C., approximately 2 ml of 95% ethanol was added followed by evaporation in vacuo with warming. After evaporation to dryness, 2 ml of water was added and concentration was done under vacuum with warming. Sufficient 0.9% sodium chloride solution was added to yield the final volume of approximately 25 ml and uniform dispersion accomplished in an ultrasonic bath.

The dispersion was administered to beagle dogs as follows. Four beagle dogs ranging in weight from 11.6-13.5 kg were used in this study. The dogs had been previously tracheostomized. The dogs were assigned to either group A, and treated with a control preparation of 0.5 mg/ml of leuprolide acetate in 0.9% sodium chloride solution, or to group B, and treated with the surfactant preparation having the composition described in Table 1.

Animals were dosed by the intrapulmonary route via a catheter inserted in a permanent stoma near the bifurcation of the major bronchi. The details of dose and volume are given in Table 2.

TABLE 2

| GROUP | PREPARATION | DOG # | DOG WEIGHT (kg) | DOSE VOLUME (ml) | DOSE OF LEUPROLIDE (mg) |
|---|---|---|---|---|---|
| A | SALINE CONTROL | 1 | 13.5 | 6 | 3 |
|  | SALINE CONTROL | 2 | 11.6 | 6 | 3 |
| B | SURFACTANT | 1 | 12.5 | 6 | 3 |
|  | SURFACTANT | 2 | 13.0 | 6 | 3 |

Blood samples (3 ml each from the jugular) were collected at 0, 5, 15, 30 and 45 minutes and at 1, 2 and 4 hours after dosing. The samples were centrifuged, the resulting plasma was frozen and analyzed for leuprolide by radioimmunoassay.

The results are shown in Table 3 and the Figure. In Table 3, AUC refers to the area under a curve generated by plotting plasma concentration versus time for the period 0-4 hours. In the Figure, dogs 1 ("D1) and 2 ("D2") were treated with the control leuprolide/saline preparation, as described above, and dogs 3 ("D3") and 4 ("D4") were treated with the leuprolide/surfactant preparation as described above. Plasma levels of leuprolide as a function of time are graphically depicted in the Figure with points plotted on a separate plot for each dog as follows: boxes for D1; pluses for D2; diamonds for D3; and triangles for D4.

TABLE 3

| PLASMA CONCENTRATION OF LEUPROLIDE (ng/ml) | | | | | | | | | MEAN AUC |
|---|---|---|---|---|---|---|---|---|---|
| GROUP | DOG | MINUTES POST ADMINSTRATION | | | | | | | |
| | | 5 | 15 | 30 | 45 | 60 | 120 | 240 | |
| A | 1 | 17.3 | 37.3 | 65.7 | 71.8 | 88.8 | 97.5 | 73.7 | |
| | 2 | 17.2 | 55.3 | 145.8 | 169.1 | 312.1 | 122.7 | 36.2 | 24824 +/-5639 |
| B | 1 | 61.4 | 372.7 | 368.0 | 285.8 | 256.3 | 100.2 | 27.8 | |
| | 2 | 510.8 | 1015.6 | 561.7 | 288.1 | 280.7 | 121.3 | 39.2 | 44144 +/-8927 |

These data demonstrate three conclusions. First, the time to peak plasma level is much shorter in the dogs dosed with a pulmonary surfactant drug vechicle. The time to peak is approximately 15 minutes in the surfactant delivery system treated animals compared with approximately 60-90 minutes in the animals treated with the saline/drug control. Second, the peak plasma level is approximately three to five fold higher in the dogs treated with the drug in the surfactant vehicle compared to those treated with the drug in saline solution as control. Third, the area under the curve through 4 hours is approximately 78% larger in the dogs treated with the surfactant delivery system compared to those treated with the drug in saline control.

Example 4

A similar process to incorporate vasopressin (8-Arg) at a concentration of 0.5 μg/ml of dispersion was carried out using the quantities of other ingredients and the same dispersion techniques as described in Example 3.

EXAMPLE 5

Polypeptides having sequences as set forth in Table 4 may be synthesized for use according to the present invention by assembly on a resin support with stepwise solid phase synthesis (starting with the carboxy terminal residue) according to the general procedure described in Barany et al., The Peptides, 2, Gross et al., eds., Academic Press, New York, 1284 (1980) on an Applied Biosystems Synthesizer, Model 430A. Protected amino acids may be coupled in a stepwise manner to the resin support by preformed symmetric anhydride chemistry, except in the case of arginine addition, wherein the DCC/HOBT protocol [Konig et al., Chem. Beer., 103, 788-798 (1970)] may be employed.

## TABLE 4

NH$_2$-Tyr-Ile-Pro-Cys-Phe-Pro-Ser-Ser-
Leu-Lys-Arg-Leu-Leu-Ile-COOH

NH$_2$-terminal amino acid residues of canine SAP-Val

NH$_2$-Leu-Ile-Pro-Cys-Cys-Pro-Val-Asn-
Ile-Lys-Arg-Leu-Leu-Ile-Val-Val-Val-
Val-Val-Val-Val-Val-COOH

NH$_2$-terminal amino acid residues of bovine SAP-Val

NH$_2$-Phe-Pro-Ile-Pro-Ile-Pro-Tyr-Cys-
Trp-Leu-COOH

NH$_2$-terminal amino acid residues of human SAP(Phe) excpt that, at position 5, Ile was selected rather than Leu.

NH$_2$-Phe-Pro-Ile-Pro-Leu-Pro-Tyr-Cys-
Trp-Leu-Cys-Arg-Ala-Leu-Ile-Lys-Arg-
Ile-Gln-Ala-Met-Ile-Pro-Lys-Gly-Ala-
Leu-Arg-Val-Ala-Val-Ala-Gln-Val-Cys-
Arg-Val-Val-Pro-Leu-Val-Ala-Gly-Gly-
Ile-Cys-Gln-Cys-Leu-Ala-Glu-Arg-Tyr-
Ser-Val-Ile-Leu-Leu-Asp-Thr-COOH

NH$_2$-terminal end of human SAP(Phe)

G I P C C P V H L K R L L I V V V V V
L I V V V I V G A L L M G L H M S Q K H
T E M V L E M S I G A P E A Q Q R L A L

Human SAP[Val(1-60)]

Leu-Ile-Pro-Cys-Cys-Pro-Val-His-Leu-Lys-     Human SAP[Val(1-31)]
Arg-Leu-Leu-Ile-Val-Val-Val-Val-Val-Val-
Leu-Ile-Val-Val-Val-Ile-Val-Gly-Ala-Leu-Leu

Phe-Pro-Ile-Pro-Leu-Pro-Tyr-Cys-Trp-Leu-     Human SAP[Phe(1-78)]
Cys-Arg-Ala-Leu-Ile-Lys-Arg-Ile-Gln-Ala-
Met-Ile-Pro-Lys-Gly-Ala-Leu-Arg-Val-Ala-
Val-Ala-Gln-Val-Cys-Arg-Val-Val-Pro-Leu-
Val-Ala-Gly-Gly-Ile-Cys-Gln-Cys-Leu-Ala-
Glu-Arg-Tyr-Ser-Val-Ile-Leu-Leu-Asp-Thr-
Leu-Leu-Gly-Arg-Met-Leu-Pro-Gln-Leu-Val-
Cys-Arg-Leu-Val-Leu-Arg-Cys-Ser

Leu-Arg-Val-Ala-Val-Ala-Gln-Val-Cys-Arg-     Human SAP[Phe(27-78)]
Val-Val-Pro-Leu-Val-Ala-Gly-Gly-Ile-Cys-
Gln-Cys-Leu-Ala-Glu-Arg-Tyr-Ser-Val-Ile-
Leu-Leu-Asp-Thr-Leu-Leu-Gly-Arg-Met-Leu-
Pro-Gln-Leu-Val-Cys-Arg-Leu-Val-Leu-Arg-
Cys-Ser

Tyr-Ser-Val-Ile-Leu-Leu-Asp-Thr-Leu-Leu-     Human SAP[Phe(53-78)]
Gly-Arg-Met-Leu-Pro-Gln-Leu-Val-Cys-Arg-
Leu-Val-Leu-Arg-Cys-Ser

EP 0 335 133 A2

Human SAP[Val(1-41)]

Gly-Ile-Pro-Cys-Cys-Pro-Val-His-Leu-Lys-
Arg-Leu-Leu-Ile-Val-Val-Val-Val-Val-Val-
Leu-Ile-Val-Val-Val-Ile-Val-Gly-Ala-Leu-
Leu-Met-Gly-Leu-His-Met-Ser-Gln-Lys-His-Thr

Human SAP[Val(12-31)]

Leu-Leu-Ile-Val-Val-Val-Val-Val-Val-Leu-
Ile-Val-Val-Val-Ile-Val-Gly-Ala-Leu-Leu

Human SAP[Val(21-41)]

Leu-Ile-Val-Val-Val-Ile-Val-Gly-Ala-Leu-
Leu-Met-Gly-Leu-His-Met-Ser-Gln-Lys-His-Thr

Human SAP[Val(-23 to -1)]

Asp-Val-Gly-Ser-Lys-Glu-Val-Leu-Met-Glu-
Ser-Pro-Pro-Asp-Tyr-Ser-Ala-Ala-Pro-Arg-
Gly-Arg-Phe

Human SAP[Phe -57 to -29)]

Lys-Ser-Arg-Gln-Pro-Glu-Pro-Glu-Gln-Glu-
Pro-Gly-Met-Ser-Asp-Pro-Leu-Pro-Lys-Pro-
Leu-Arg-Asp-Pro-Leu-Pro-Asp-Pro-Leu

Human SAP[Phe(-28 to -1)]

Leu-Asp-Lys-Leu-Val-Leu-Pro-Val-Leu-Pro-
Gly-Ala-Leu-Gln-Ala-Arg-Pro-Gly-Pro-His-
Thr-Gln-Asp-Leu-Ser-Glu-Gln-Gln

Although the present invention has been described herein in terms of preferred embodiment, it is expected that various modifications and improvements will occur to those skilled in the art upon consideration of the present invention. For example, administration according to the present invention of drugs may be made by pulmonary delivery through nebulization, aerosolization or direct lung instillation. The drug administered may include those drugs which act specifically on the lung, those which may be better absorbed from the lung due to lack of absorption via the oral route of administration, or those which may be destroyed or inactivated when given orally.

It is anticipated that pulmonary surfactant may be concurrently administered, according to the present invention, with certain drug classes which may be pulmonary toxic and where this toxicity is related to: modification or destruction of native pulmonary surfactant; or, alternatively, to actions which decrease the amount available to the lung, either from loss through the lung; or, alternatively, to a reduction in the amount synthesized or secreted in the Type II epithelial cell.

Various drug classes may be incorporated in a surfactant composition according to the present invention to provide for improving the peak plasma levels in systemic blood where the drug is readily absorbed in the air-blood interface at the alveoli.

It is expected that the present invention provides for increased area under the time vs. blood concentration curve over equivalent periods of time due to better blood levels of the drug as well as the potential for less first pass hepatic metabolism due to the route of absorption.

Drugs categories useful in the process of the present invention, and examples of potential application include without limitation:

(a) hormonal drugs, including analog agonists and antagonists, such as leuprolide and vasopressin, steriod hormones;

(b) anti-infective agents to treat either systemic or specific pulmonary related microbial, viral or mycoplasma-related diseases, including: erthromycin, and salts and analogs; cephem-antibiotics, including the subgroup known as penicillins but generally all beta-lactam drugs which are antibiotic in nature; cycloserine and salts and/or analogs thereof; isoniazide, rifamycin, capreomycin, etionamide or other anti-tuberculosis drugs; pentamidine, trimethoprin, sulfmethoxazole and such other drugs for the treatment of specific pulmonary diseases, especially those associated with the disease known as AIDS or ARC, such as Pneumocystis carinii, where the direct administration of such drugs to the lung may offer better activity;

(c) anti-mucolytic agents, such as acetylcysteine or guiafesin, which benefit from direct lung administration because they act on the lung to facilitate clearing mucus from the lung;

(d) anti-cancer or antineoplastic agents, such as drugs including adriamycin or lomustine which benefit from direct lung adminstration due to a localized site specific administration to avoid first pass metabolic effects or systemic toxic effects which may be minimized so as to make them less toxic, reduce the amount necessary to affect an active dose or where poor absorption may be characteristic;

(e) certain of the beta-adrenergic drugs used specifically as bronchodilators where their activity on specific beta-adrenergic receptors in the bronchial region may increase the rate of onset of action due to high local or systemic levels in a shorter period of time [This may be particularly important in emergency situations where a rapid onset of action would be of benefit. Such drugs may include, without restriction, isoproterenol, metaprolol or albuterol. Without respect to whether a larger absorbed dose or a more rapid onset of action is desired, the activity of such drugs may be improved.]; and

(f) certain of the thrombolytic or anti-coagulant drugs which could be used for immediate or longer term therapy of thrombolytic diseases of the lung or other organs. Such drugs would include urokinase, streptokinase, streptokinase-plasminogen complex, tissue plasminogen activator and heparin.

Accordingly, it is intended that all such modifications and improvements come within the scope of the following claims.

## Claims

1. A composition for pulmonary drug delivery comprising:
a phospholipid;
a pulmonary surfactant protein; and
a drug to be administered combined with said protein and phospholipid.

2. The composition as recited in claim 2 wherein said composition comprises:
50-95% phospholipid;
0-15% neutral lipid;
0.15% fatty acid;
0.01-10% protein; and
0.1-3% chloesterol.

3. The composition as recited in claim 3 further comprising a pharmaceutical excipient.